Europäisches Patentamt

European Patent Office

Office européen des brevets

(19)

(11) EP 0 953 341 A2

(12) EUROPEAN PATENT APPLICATION

(43) Date of publication:
03.11.1999 Bulletin 1999/44

(51) Int Cl.$^6$: A61K 7/48, A61K 35/78

(21) Application number: 99400437.2

(22) Date of filing: 23.02.1999

(84) Designated Contracting States:
AT BE CH CY DE DK ES FI FR GB GR IE IT LI LU MC NL PT SE
Designated Extension States:
AL LT LV MK RO SI

(30) Priority: 24.02.1998 JP 5890498

(71) Applicant: SHISEIDO COMPANY, LTD.
Chuo-ku Tokyo (JP)

(72) Inventors:
• Yoshida, Yuzo, c/o Shiseido Res. Center
  Yokohama-shi, Kanagawa 223-8553 (JP)
• Ota, Masahiro, c/o Shiseido Res. Center
  Yokohama-shi, Kanagawa 223-8553 (JP)

(74) Representative: Rinuy, Santarelli
14, avenue de la Grande Armée
75017 Paris (FR)

(54) Antiplasmin agent

(57) An antiplasmin agent containing a plant belonging to Pyrolaceae or the extract thereof in a carrier therefor, having the excellent effect of improving or preventing skin roughening and chapping.

EP 0 953 341 A2

**Description**

BACKGROUND OF THE INVENTION

1. Field of the Invention

The present invention relates to an antiplasmin agent. More specifically, it relates to an antiplasmin agent having the effect of improving or preventing skin roughing and chapping caused by, for example, dryness, cleansers, in addition to various types of skin diseases such as contact type dermatitis, psoriasis, pemphigus vulgaris, and congenital herpes, in which a change in activity of the plasmin or plasminogen activator is observed at the diseased location.

2. Description of the Related Art

Various types of therapeutic agents, skin external application agents, cosmetics, etc. having the effect of improving or treating skin diseases or rough skin have been heretofore known. These conventional medicines and cosmetics etc. contain, as the effective ingredients, amino acids or polysaccharides, lipids, extracts, etc. having an anti-inflammatory activity or a high moisturizing effect due to their superior capability of preventing inflammation of the skin or disappearance of the moisture in the corneal layer. However, in any event, the effect of improving or preventing rough skin has not necessarily been sufficient, and therefore, development of antiplasmin agents having more superior medicinal effect has been desired.

On the other hand, it has been considered that in the normal process of cornification of the skin, the protease in the epidermal cells plays an important role (Ogawa H., Yoshiike T.; *Int. J. Dermatol;* 23, 1984), and it has been made clear that the change in activity of the protease such as plasmin and plasminiogen activator (PA) has a deep involvement in the formation of lesions of various types of skin diseases in recent years. For example, it has been reported that there is strong PA activity present at parakeratosis locations of diseased skin (Haustein; *Arch. Klin. Exp. Dermatol;* 234, 1969) and that PA has been extracted from psoriasis scales using a high concentration salt solution (Fraki. Hopsu-Havu; *Arch. Dermatol. Res;* 256, 1976). Plasminogen activator is a protease which specifically acts on a plasminogen, a precursor of plasmin, and converts them to active form, plasmin. Further, in pemphigus vulgaris, the plasminogen activator produced in large amounts in the epidermal cells convert the plasminogen present outside the cells to plasmin. This plasmin digests the intercellular connective substances, whereby a tissue fluid is stored between the cells and intraepidermal vesicles are formed as has been made clear in *in vitro* experiments (Morioka S.; *J. Invest. Dermatol;* 76, 1981).

SUMMARY OF THE INVENTION

In view of the above situation, the present inventors thought that a plasmin inhibitor would be effective for improvement of various skin diseases, rough skin, chaffing, etc. and investigated the antiplasmin activity of a wide variety of substances. As a result, we found that extracts from plants of the *Pyrolaceae* have a superior inhibition activity against plasmin.

Accordingly, the objects of the present invention are to eliminate the above-mentioned problems in the prior art and to provide an antiplasmin agent having the superior effects of improving or preventing skin roughing and chapping.

In accordance with the present invention, there is provided an antiplasmin agent comprising a plant of the *Pyrolaceae* or the extract, as an effective component.

DESCRIPTION OF THE PREFERRED EMBODIMENTS

As far as the present inventors know, *Pyrolaceae* plants have an antiinflammatory effect and effect of preventing or improving rough skin (Japanese Unexamined Patent Publication (Kokai) No. 3-190809), an antibacterial activity on bacteria causing gum diseases (Japanese Unexamined Patent Publication (Kokai) No. 4-5222), an effect of improving freckles (Japanese Unexamined Patent Publication (Kokai) No. 4-279511), a testosteron 5a-reductase inhibition activity (Japanese Unexamined Patent Publication (Kokai) No. 5-17365), etc. and have applications as skin external application agents, cosmetics, and oral compositions, but there have not been any reports on an antiplasmin activity thereof until now.

The construction of the present invention will be explained in detail below.

The plants belonging to the *Pyrolaceae* used in the present invention includes, for example, *Pyrola japonica Klenze, Pyrola incarnata Fischer, Pyrola renifolica Maxim., Pyrola rotundifolia L. subsp. chinensis Andr.* The *Pyrola rotundifolia L. subsp. chinensis Andr.* is mainly found in China and is a different species from the Japanese *Pyrola*

*japonica Klenze,* but *Pyrola japonica Klenze* and *Pyrola incarnata Fischer* are known by the Chinese name *Luticao.* In the present invention, *Pyrola japonica Klenze* is particularly preferably used among these from the viewpoint of the strong plasmin inhibiting activity or ease of acquisition domestically.

The antiplasmin agent according to the present invention may be obtained by drying, then pulverizing to a powder, any of the entire plant, leaves, flower, or roots of the *Pyrola japonica Klenze* or immersing the same together with an extraction solution or heat refluxing the same, then filtering, but it is preferable to use an extract from the viewpoints of useability, easy formulation, etc.

As the extraction solution of the *Pyrola japonica Klenze* plant, any conventional solvent for extraction may be used. For example, alcohols such as methanol, ethanol; and organic solvents such as acetone, ethyl acetate; and water may be used alone or in any mixture thereof. Further, it is also possible to use the above solvents, followed by purifying the extract with, for example, partition or chromatography and use the resultant product.

The antiplasmin agent according to the present invention is mainly used, as an external application agent usually with a carrier therefor. The amount of the *Pyrola japonica Klenze* plant or the extract thereof formulated in the composition is preferably 0.005 to 20.0% by weight, and more preferably 0.01 to 10.0% by weight, as the dried product in the total weight of the composition. If the amount is less than 0.005% by weight, the advantageous effects of the present invention are not sufficiently exhibited, whereas if the amount is more than 20.0% by weight, the formulation becomes difficult. Further, even if over 10.0% by weight is formulated, the further improvement in the advantageous effect cannot be, expected. As the carrier, any conventional carrier may be used in the present invention.

The antiplasmin agent of the present invention may optionally contain, in addition to the essential component, the other components normally used for skin external application agents such as cosmetics, pharmaceuticals. Examples of such optional components are whitening agents, moisturizers, antioxidants, oil ingredients, ultraviolet absorbers, surfactants, thickeners, alcohols, powder ingredients, coloring matter, aqueous ingredients, water, various skin nutrients, etc.

In addition, it is possible to use, for example, metal chelating agents such as, disodium edetate, trisodium edetate, sodium citrate, sodium polyphosphate, sodium metaphosphate, gluconic acid; caffeine, tannin, verapamil, tranexamic acid, and their derivatives glabridin, various the herb extracts; the other medicines such as tocopherol acetate, glycyrrhizic acid, and their derivatives or salts; whitening agents such as vitamin C, magnesium ascorbate phosphate, ascorbate glucoside, arbutin, kojic acid; saccharides such as glucose, fructose, mannose, sucrose, trehalose.

When the antiplasmin agent of the present invention is used, as an external application agent, for example, it may be in any conventional form such as, for example, an ointment, cream, emulsion, lotion, pack, bath agent, etc. and other forms conventionally used for skin external application agents. The form is not particularly critical.

EXAMPLES

The present invention will now be further illustrated in detail by, but is by no means limited to the following Examples. The formulation amounts in the Examples are based on % by weight.

First, the test methods and evaluation criteria of the antiplasmin activity and effect of skin improvement of *Pyrola japonica Klenze* according to the present invention will now be explained.

1. Test of Plasmin Inhibition Activity

(1) Preparation of Sample

50 g (dried weight) of the entire plant of the *Pyrola japonica Klenze* in the flowering period were immersed in five times volumes of 50% ethanol at room temperature for 1 week. The extract was then concentrated to dryness. The solid product was dissolved in dimethylsulfoxide (DMSO) to prepare a 3% solution. This was used for the following Experiments.

(2) Measurement of Plasmin Inhibition Activity

The rate of inhibition % was determined by the fibrin flat plate method. That is, 6 ml of a Veronal buffer (25 mmol/L barbital sodium solution containing 0.125 mol/L NaOH, pH 7.4) containing 1.0% of fibrinogen (plasminogen removed) was poured into a 9 cmφ petri dish. 0.2 ml of 1.0 mol/L $CaCl_2$ and 0.1 ml of 25U/ml thrombin were added thereto, gently mixed, and allowed to stand for 1 hour. A mixture of 5U/ml plasmin and the test substance mixed in a ratio of 29:1 was allowed to stand at 37°C for 10 minutes, then 20 µl of the mixture was added on a plate formed by fibrinogen's conversion to fibrin. And, then, was further allowed to stand at 37°C for 18 hours. As a control, the same procedure was followed using DMSO, instead of the test sample, then the area of the dissolution circle formed by the dissolution of the fibrin was measured. The plasmin inhibition ratio was determined by the following mathematical formula (I):

$$\text{Inhibition ratio (\%)} = \{1-(\text{area of dissolution circle of test sample})/(\text{area of dissolution circle of control})\} \times 100 \qquad (I)$$

The results are shown in Table 1.

Further, as a Reference Example, the same test as above was conducted on an ethanol extract of *Phellodendron amurense Ruprecht,* a plant considered to have an anti-inflammatory and stringent action and already known to have applications against rough skin. The results are shown, together, in Table 1.

Table 1

| (Final concentration) | | |
|---|---|---|
| | Final Concentration of sample added (wt%) | Plasmin inhibition ratio (%) |
| *Pyrola japonica Klenze* | 0.1 | 55.6 |
| | 0.01 | 22.6 |
| *Phellodendron amurense Ruprecht* | 0.1 | 17.6 |
| | 0.01 | 5.9 |

As will be understood from Table 1, the *Pyrola japonica Klenze* extract has a superior plasmin inhibition activity, compared with the *Phellodendron amurense Ruprecht* extract.

2. Actual Usage Tests (Test of Improvement of Razor Burn (Sycosis) and Test of Prevention of Rough Skin in Winter)

The effect of use of the external application agent composition according to the present invention on the outer skin was evaluated from the effect of improvement of razor burn and skin irritation and the prevention of skin roughness in the winter by the following evaluation methods.

Note that, as the sample, as shown in Table 2, 2 types of lotions having different concentrations of the 50% ethanol extract of the entire plant of *Pyrola japonica Klenze* in the flowering period were used as the products of the present invention, while a lotion containing a 50% ethanol extract of *Phellodendron amurense Ruprecht,* already known to have applications on rough skin, and a lotion not containing *Pyrola japonica Klenze extract* or *Phellodendron amurense Ruprecht* extract were used as the comparative products. The results are shown, together, in Table 2.

(1) Improvement of Razor Burn

40 male panelists with razor burn were divided into 4 groups of 10 each, the products of the present invention or comparative products shown in Table 2 were applied immediately after shaving, and the effect on razor burn was judged. The judgement criteria and evaluation were as follows:

Judgement Criteria of Razor Burn Improving Effects

Very effective: Razor burn disappears.
Effective: Razor burn becomes weaker.
Somewhat effective: Razor burn becomes somewhat weaker.
Ineffective: No change observed in razor burn.

Evaluation of Razor Burn Improving Effects

Excellent:  80% or more ratio of test subjects indicating very effective, effective, or somewhat effective (efficacy).
Good:  50% to less than 80% ratio of test subjects indicating very effective, effective, or somewhat effective (efficacy).
Fair:  30% to less than 50% ratio of test subjects indicating very effective, effective, or somewhat effective (efficacy).
Poor:  Less than 30% ratio of test subjects indicating very effective, effective, or somewhat effective (efficacy).

(2) Skin Irritation

When judging the rate of improvement of razor burn, the skin irritations of the products of the present invention and comparative products were judged and evaluated. The evaluation criteria were as follows:

Evaluation of Skin Irritation

Excellent:  0% test subjects observing burning sensation on skin.
Good:  Less than 5% test subjects observing skin irritation.
Fair:  Less than 10% test subjects observing skin irritation.
Poor:  10% or more test subjects observing skin irritation.

Table 2

| Sample | Invention products | | Comparative products | |
|---|---|---|---|---|
| | 1 | 2 | 1 | 2 |
| 50% ethanol extract of *Pyrola japonica Klenze* | 3.0 | 1.0 | - | - |
| 50% ethanol extract of *Phellodendron amurense Ruprecht* | - | - | 3.0 | - |
| Glycerol | 1.0 | 1.0 | 1.0 | 1.0 |
| 1,3-Butylene glycol | 4.0 | 4.0 | 4.0 | 4.0 |
| Ethanol | 7.0 | 7.0 | 7.0 | 7.0 |
| Polyoxyethylene (20 mol) oleyl alcohol | 0.5 | 0.5 | 0.5 | 0.5 |
| Purified water | Bal. | Bal. | Bal. | Bal. |
| Razor burn improving effect | Excl. | Fair | Fair | Poor |
| Skin irritation | Excl. | Excl. | Excl. | Excl. |

As clear from Table 2, the lotion of product 2 of the present invention containing 1.0% by weight of *Pyrola japonica Klenze* extract exhibited an equal efficacy as the lotion of comparative product 1 containing 3.0% by weight of *Phellodendron amurense Ruprecht* extract, while the lotion of product 1 of the present invention containing 3.0% by weight of *Pyrola japonica Klenze* extract exhibited a superior effect of improvement on razor burn, compared with comparative product 1. Further, no skin irritation could be observed at that time.

(3) Prevention of Rough Skin in Winter

Replicas of the facial skin of female panelists particularly suffering from rough skin in the winter were taken in the autumn (October) and the skin conditions were observed under a microscope (17X). The skins were ranked in accordance with the judgement criteria shown below. 40 women ranked 4 or 5 were selected and divided into 2 groups of 20 each. The lotions of the product 1 of the present invention and comparative product 1 or the product 2 of the present invention and comparative product 2 were applied in the winter (December) twice a day to halves of the faces, right and left, of 20 women each for 4 weeks, then replicas were taken and the skin conditions were ranked again according to the above judgement criteria. The difference in skin ranks before and after the test was found for each panelist and the rough skin preventing effect in the winter was judged. The results are shown in Table 3.

Skin Rank and Judgement Criteria

1: Disappearance of sulcus cutis and crista cutis and scaling over wide area observed.
2: Unclear sulcus cutis and crista cutis and scaling observed.
3: Sulcus cutis and crista cutis observed, but flat.
4: Clear sulcus cutis and crista cutis.
5: Clear, full range of sulcus cutis and crista cutis.

Table 3

| Difference in skin rank | Invention product 1 | Invention product 2 | Comparative product 1 | Comparative product 2 |
|---|---|---|---|---|
| -3 | 0 | 0 | 0 | 1 |
| -2 | 0 | 0 | 4 | 6 |
| -1 | 5 | 8 | 10 | 9 |
| 0 | 13 | 12 | 6 | 4 |
| +1 | 2 | 0 | 0 | 0 |

As will be understood from Table 3, the lotions of the products according to the present invention exhibit an advantageous effect of prevention of rough skin in the winter, believed to be caused by dryness etc., compared with the lotions of the comparative products.

Example 1: Cream

| Recipe | Blend ratio |
|---|---|
| Stearic acid | 5.0 |
| Stearyl alcohol | 4.0 |
| Isopropyl myristate | 18.0 |
| Glyceryl monostearic ester | 3.0 |
| Propylene glycol | 10.0 |
| 70% Ethanol extract of *Pyrola japonica Klenze* | 0.05 |
| Potassium hydroxide | 0.2 |
| Tranexamic acid | 0.5 |
| Sodium bisulfite | 0.01 |
| Preservative | q.s. |
| Perfume | q.s. |
| Ion exchange water | Bal. |

Preparation Method

The propylene glycol, *Pyrola japonica Klenze* ethanol extract, and potassium hydroxide were added to the ion exchange water, then heated and held at 70°C (aqueous phase). The rest of the ingredients were mixed, heated to melt, and held at 70°C (oil phase). The oil phase was gradually added to the aqueous phase. After the entire amount was added, the mixture was allowed to stand at that temperature for a while to cause the reaction. Thereafter, the mixture was uniformly emulsified by a homomixer, then cooled to 30°C while stirring well.

Example 2: Cream

| Recipe | Blend ratio |
|---|---|
| Stearic acid | 2.0 |
| Stearyl alcohol | - 7.0 |
| Hydrogenated lanolin | 2.0 |
| Squalane | 5.0 |
| 2-Octyldodecyl alcohol | 6.0 |
| Polyoxyethylene (25 mol) cetyl alcohol ether | 3.0 |
| Glyceryl monostearic ester | 2.0 |
| Propylene glycol | 5.0 |
| Methanol extract of *Pyrola japonica Klenze* | 0.05 |
| Tranexamic acid | 0.2 |
| Sodium bisulfite | 0.03 |
| Ethyl paraben | 0.3 |
| Perfume | q.s. |
| Ion exchange water | Bal. |

Preparation Method

The propylene glycol was added to the ion exchange water, then heated and held at 70°C (aqueous phase). The rest of the ingredients were mixed, heated to melt, and held at 70°C (oil phase). The oil phase was added to the aqueous phase and the mixture was preemulsified, then was uniformly emulsified by a homomixer, then cooled to 30°C while stirring well.

Example 3: Cream

| Recipe | Blend ratio |
|---|---|
| Solid paraffin | 5.0 |
| Beeswax | 10.0 |
| Vaseline | 15.0 |
| Liquid paraffin | 41.0 |
| Glyceryl monostearate ester | 2.0 |
| Polyoxyethylene (20 mol) sorbitan monolaurate ester | 2.0 |
| Powder soap | 0.1 |
| N-methyl-trans-4-amino methyl cyclohexane carboxamide | 1.0 |
| Sodium borate | 0.2 |
| 70% Methanol extract of *Pyrola japonica Klenze* | - 0.1 |
| Sodium bisulfite | 0.03 |
| Ethyl paraben | 0.3 |
| Perfume | q.s. |
| Ion exchange water | Bal. |

Preparation Method

The powder soap and sodium borate were added to the ion exchange water, then heated and held at 70°C (aqueous phase). The rest of the ingredients were mixed, heated to melt, and held at 70°C (oil phase). The oil phase was gradually added to the aqueous phase while stirring for the reaction. Next, the resultant mixture was uniformly emulsified by a homomixer, then cooled to 30°C while stirring well.

Example 4: Emulsion

| Recipe | Blend ratio |
|---|---|
| Stearic acid | 2.5 |
| Cetyl alcohol | 1.5 |
| Vaseline | 5.0 |
| Liquid paraffin | 10.0 |
| Polyoxyethylene (10 mol)monooleic ester | 2.0 |
| Polyethylene glycol 1500 | 3.0 |
| Triethanolamine | 1.0 |
| Carboxyvinyl polymer (tradename: Carbopol 941, B.F. Goodrich Chemical Co.) | 0.05 |
| Ethyl acetate extract of *Pyrola japonica Klenze* | 0.03 |
| Sodium bisulfite | 0.01 |
| Ethyl paraben | 0.3 |
| Perfume | q.s. |
| Ion exchange water | Bal. |

Preparation Method

The carboxyvinyl polymer was dissolved in a small amount of the ion exchange water (phase A). The polyethylene glycol 1500 and the triethanolamine were added to the remaining ion exchange water, heated to dissolve, and held at 70°C (aqueous phase). The other ingredients were mixed, heated to melt, and held at 70°C (oil phase). The oil phase was added to the aqueous phase and the mixture was preliminarily emulsified, then phase A was added and the mixture emulsified uniformly by a homomixer. After emulsification, the emulsion was cooled to 30°C while stirring well.

Example 5: Emulsion

| Recipe | Blend ratio |
|---|---|
| Microcrystalline wax | 1.0 |
| Beeswax | 2.0 |
| Lanolin | 20.0 |
| Liquid paraffin | 10.0 |
| Squalane | 5.0 |
| Sorbitan sesquioleic ester | 4.0 |
| Polyoxyethene (20 mol) sorbitan monooleic ester | 1.0 |
| Propylene glycol | 7.0 |
| 30% Butanol extract of *Pyrola japonica Klenze* | 2.0 |
| Tranexamic acid | 1.0 |
| Sodium bisulfite | 0.01 |
| Ethyl paraben | 0.3 |
| Perfume | q.s. |
| Ion exchange water | Bal. |

Preparation Method

Propylene glycol was added to the ion exchange water and heated and held at 70°C (aqueous phase). The rest of the ingredients were mixed and heated to melt and held at 70°C (oil phase). The oil phase was gradually added to the aqueous phase while stirring. The mixture was uniformly emulsified by a homomixer, then was cooled to 30°C while stirring well.

Example 6: Jelly

| Recipe | Blend ratio |
|---|---|
| 95% Ethyl alcohol | 10.0 |
| Dipropylene glycol | 15.0 |
| Polyoxyethylene (50 mol) oleyl alcohol. ether | 2.0 |
| Carboxyvinyl polymer (tradename: Carbopol 940, B.F. Goodrich Chemical Co.) | 0.05 |
| Sodium hydroxide | 0.15 |
| L-arginine | 0.1 |
| 30% Ethanol extract of *Pyrola japonica Klenze* | 1.0 |
| Sodium bisulfite | 0.01 |
| Ethyl paraben | 0.3 |
| Perfume | q.s. |
| Ion exchanqe water | Bal. |

Preparation Method

The Carbopol 940 was uniformly dissolved in ion exchange water. On the other hand, the *Pyrola japonica Klenze* extract, polyoxyethylene (50 mol) oleyl alcohol ether were dissolved in 95% ethanol and added to the aqueous phase. Next, the rest of the ingredients were added, then the resultant mixture was neutralized and thickened with sodium hydroxide and L-arginine.

Example 7: Pack

| Recipe | Blend ratio |
|---|---|
| (Phase A) | |
| Dipropylene glycol | 5.0 |
| Polyoxyethylene (60 mol) hydrogenated castor oil | 5.0 |
| (Phase B) | |
| Olive oil | 5.0 |
| Tocopherol acetate | 0.2 |
| Ethyl paraben | 0.2 |
| Perfume | 0.2 |
| (Phase C) | |
| Sodium bisulfite | 0.03 |
| Polyvinyl alcohol (degree of saponification 90, degree of polymerization 2000) | 13.0 |
| Ethyl alcohol | 7.0 |
| 50% 1.3-Butylene glycol extract of *Pyrola japonica Klenze* | 1.0 |
| Purified water | Bal. |

Preparation Method

Phase A and phase B were uniformly dissolved, then phase B was added to phase A to solubilize it. Next, phase C in which the *Pyrola japonica Klenze* extract was dispersed was added thereto, then the resultant product was filled.

Example 8: Foundation

| Recipe | Blend ratio |
|---|---|
| Talc | 43.1 |
| Kaolin | 15.0 |
| Sericite | 10.0 |
| Zinc oxide | 7.0 |
| Titanium dioxide | 3.8 |
| Yellow iron oxide | 2.9 |
| Black iron oxide | 0.2 |
| Dried powder of *Pyrola japonica Klenze* | 0.05 |
| Squalane | 8.0 |
| Isostearic acid | 4.0 |
| Monooleate POE sorbitan | 3.0 |
| Isocetyl octanate | 2.0 |
| Preservative | q.s. |
| Perfume | q.s. |

Preparation Method

The entire plant of sufficiently dried *Pyrola japonica Klenze* was finely pulverized by a pulverizer and passed through a # 30 (500 µm) sieve. The resultant powder and the powder ingredients from the talc to the black iron oxide were sufficiently mixed by a blender, then the other ingredients were added and the resultant mixture was kneaded well and filled into a container for molding.

As explained above, according to the present invention, it is possible to provide an antiplasmin agent having a superior effect of improving and preventing various skin diseases accompanied with rough skin and rough skin, chaffing, etc. of healthy persons.

**Claims**

1. An antiplasmin agent comprising a plant belonging to *Pyrolaceae* or the extract thereof, as an effective component.

2. An antiplasmin agent as claimed in claim 1, wherein the amount of the plant belonging to Pyrolaceae or the extract thereof formulated is 0.005 to 20.0% by weight, based upon the total weight of the composition.

3. An antiplasmin agent as claimed in claim 1, wherein the plant belonging to Pyrolaceae is at least one member selected from the group consisting of *Pyrola japonica Klenze, Pyrola incarnata Fischer, Pyrola renifolica Maxim.* and *Pyrola rotundifolia L. subsp. chinensis Andr.*

4. An antiplasmin agent as claimed in claim 1, wherein the effective component is derived from at least one member selected from the group consisting of the entire plant, leaves, flower or roots of the plant belonging to Pyrolaceae is.

5. An antiplasmin agent as claimed in claim 4, wherein the effective component is in the form of a powder obtained by drying and then pulverizing said at least one member of the plant.

6. An antiplasmin agent as claimed in claim 4, wherein the effective component is an extract obtained from the extraction of said at least one member of the plant with a solvent.

7. An antiplasmin agent as claimed in claim 1 in the form of an external application agent.

8. An antiplasmin agent as claimed in claim 1 in the form of a cosmetic composition or pharmaceutical composition.